# EUROPÄISCHE PATENTANMELDUNG

(11) **EP 2 893 879 A1**
(43) Veröffentlichungstag der Anmeldung: **15.07.2015**
(21) Anmeldenummer: 14150996.8
(22) Anmeldetag: 13.01.2014
(51) Int. Cl.: A61B 8/00

(54) **Überzug für einen Sonografieschallkopf, Vorrichtung und Verpackungseinheit**

(71) Anmelder: PIONIRA Medical GmbH, 69493 Hirschberg (DE)
(72) Erfinder: Wanderer, Inès, 69493 Hirschberg (DE); Borchert, Ulrich, 12435 Berlin (DE)
(74) Vertreter: Horn Kleimann Waitzhofer Patentanwälte PartG mbB

(57) **Zusammenfassung**

Die Erfindung betrifft einen Überzug (6) für einen Sonografieschallkopf zum Gebrauch unter sterilen Bedingungen. Der Überzug weist einen schlauchförmigen Basiskörper (8) auf, in dem der Sonografieschallkopf aufnehmbar ist und dessen Länge (l) vorzugsweise so groß ist, dass die offenen Stirnseite (9) aus einem sterilen Feld herausführbar ist. Der Überzug weist außerdem einen die zweite Stirnseite (10) des Basiskörpers (8) verschließenden Boden (11) auf, wobei der Boden (11) eine Ankopplungssubstanz (7) zum Ankoppeln des Sonografieschallkopfs an einen zu untersuchenden Körperbereich eines Patienten aufweist. Die Ankopplungssubstanz ist in den Boden integriert. Vorzugsweise ist der Boden (11) kissenförmig. Dadurch, dass der Boden die Ankopplungssubstanz aufweist, ist ein direkter Kontakt des Sonografieschallkopfs mit der Ankopplungssubstanz verzichtbar. Die Gefahr einer Kontamination des Patienten mit unsteriler Ankopplungssubstanz wird deutlich verrringert und eine aufwändige Reinigung des Sonografieschallkopfs von der Ankopplungssubstanz ist nicht erforderlich.

## Beschreibung

Die vorliegende Erfindung betrifft einen Überzug für einen Sonografieschallkopf, eine Vorrichtung mit einem Sonografieschallkopf und einem solchen Überzug und eine Verpackungseinheit mit einem solchen Überzug.

Die Verwendung von Sonografiegeräten unter sterilen Bedingungen findet in diversen operativen Disziplinen ihren Einsatzbereich. Speziell im Bereich der Tumorchirurgie erlangt die interoperative Darstellung des Tumors immer größere Bedeutung. Immer dort, wo ein absolut gezieltes und optimal gestaltetes Vorgehen maximale Sicherheit für den Patienten bringt, ist die Absicherung des Befundes unverzichtbar. Hierfür werden in nahezu jedem Operationssaal Sonografiegeräte eingesetzt. Zur Ankopplung des Sonografieschallkopfs eines medizinischen Sonografiegeräts an einen zu untersuchenden Körperbereich eines Patienten ist eine Ankopplungssubstanz erforderlich. Die Ankopplungssubstanz kann ein sogenanntes Ultraschallgel sein. Um unter sterilen Bedingungen ein Sonografiegerät einsetzen zu können, ist es bekannt, einen sterilen Kunststoffbeutel mit der Ankopplungssubstanz zu befüllen, in welche anschließend der Sonografieschallkopf eingetaucht wird.

Vor diesem Hintergrund besteht eine Aufgabe der vorliegenden Erfindung darin, die Applikation einer Ankopplungssubstanz an einen Sonografieschallkopf zu verbessern.

Demgemäß wird ein Überzug für einen Sonografieschallkopf eines medizinischen Sonografiegeräts vorgeschlagen. Der Überzug umfasst einen schlauchförmigen Basiskörper, in dem der Sonografieschallkopf aufnehmbar ist, und einen eine Stirnseite des Basiskörpers verschließenden Boden, wobei der Boden eine Ankopplungssubstanz zum Ankoppeln des Sonografieschallkopfs an einen zu untersuchenden Körperbereich eines Patienten aufweist.

Vorzugsweise ist der Überzug dazu eingerichtet, den Sonografieschallkopf vollständig und eine Kabelverbindung von dem Sonografieschallkopf zu einem Ultraschallgenerator des medizinischen Sonografiegeräts zumindest teilweise aufzunehmen. Dadurch, dass der Boden die Ankopplungssubstanz aufweist, ist ein direkter Kontakt des Sonografieschallkopfs mit der Ankopplungssubstanz verzichtbar. Die Gefahr einer Kontamination des Patienten mit unsteriler Ankopplungssubstanz wird deutlich verrringert und die Handhabung erleichert. Außerdem ist eine aufwändige Reinigung des Sonografieschallkopfs von der Ankopplungssubstanz nicht erforderlich. Der Arbeitsaufwand zum Reinigen des Sonografieschallkopfs durch den Gebrauch des Überzugs wird somit auf Null reduziert. Ferner ist der Überzug auch unter sterilen Bedingungen verwendbar, da der Überzug steril verpackt und geliefert wird, bzw. die Ankopplungssubstanz keinen direkten Kontakt mit dem zu untersuchenden Körperbereich des Patienten hat. Der Überzug ist vorzugsweise ein Einmalartikel oder Disposal und wird nach einmaligem Gebrauch von dem Sonografieschallkopf entfernt und entsorgt. Hierdurch werden die in der Anschaffung kostenintensiven Sonografieschallköpfe geschont, wodurch deren Einsatzzeitraum verlängerbar ist. Daraus ergibt sich eine Kostenreduktion. Unter "schlauchförmig" kann auch rohrförmig oder tütenförmig zu verstehen sein. Der Basiskörper kann einen runden oder rechteckigen Querschnitt aufweisen.

Gemäß einer Ausführungsform ist der Boden kissenförmig.

Unter "kissenförmig" ist vorliegend zu verstehen, dass die Ankopplungssubstanz von Material des Bodens in Form eines Kissens vollständig umschlossen ist. Hierdurch wird ein direkter Kontakt der Ankopplungssubstanz mit dem Patienten und zugleich mit dem Sonografieschallkopf verhindert. Der Boden kann eine plattenförmige oder linsenförmige Querschnittsgeometrie aufweisen.

Gemäß einer weiteren Ausführungsform ist die Ankopplungssubstanz in den Boden integriert.

Vorzugsweise ist die Ankopplungssubstanz nur in den Boden integriert. Das heißt, der schlauchförmige Basiskörper weist die Ankopplungssubstanz nicht auf.

Gemäß einer weiteren Ausführungsform weist der Boden eine erste Wandung und eine beabstandet von der ersten Wandung angeordnete zweite Wandung auf.

Die Wandungen können parallel zueinander angeordnet sein. Unter "beabstandet" ist vorliegend zu verstehen, dass zwischen den Wandungen ein Freiraum vorhanden ist, in dem die Ankopplungssubstanz aufgenommen ist. Alternativ können die Wandungen oder zumindest eine der Wandungen gekrümmt sein. Hierdurch kann der Boden eine gewölbte oder linsenförmige Geometrie aufweisen.

Gemäß einer weiteren Ausführungsform ist die Ankopplungssubstanz zwischen der ersten Wandung und der zweiten Wandung angeordnet.

Hierzu ist zwischen den Wandungen vorzugsweise ein Spalt vorgesehen, in dem die Ankopplungssubstanz aufgenommen ist. Der Spalt zwischen den Wandungen ist vorzugsweise luftfrei und ist vollständig mit der Ankopplungssubstanz aufgefüllt.

Gemäß einer weiteren Ausführungsform sind die Wandungen aus einem elastisch verformbaren Material gefertigt.

Dadurch, dass die Wandungen aus dem elastisch verformbaren Material gefertigt sind, ist der Sonografieschallkopf in den Boden eindrückbar und kann auf diesem hin und her bewegt werden. Das heißt, der Sonografieschallkopf kann auf der Ankopplungssubstanz hin und her gleiten. Hierdurch ist stets ein flächiger Kontakt zwischen dem Sonografieschallkopf und der Ankopplungssubstanz gewährleistet, wodurch eine hohe Bildqualität gewährleistet ist. Insbesondere sind zwischen dem Boden des Überzugs und dem Sonografieschallkopf keine Lufteinschlüsse vorhanden.

Gemäß einer weiteren Ausführungsform ist das elastisch verformbare Material eine Polyethylenfolie oder eine Silikonelastomerfolie.

Alternativ kann das elastisch verformbare Material ein beliebig anderes elastisch verformbares Kunststoffmaterial sein. Vorzugsweise weisen der Basiskörper und die Wandungen eine Dicke von kleiner als 0,5 Millimeter, weiter bevorzugt von kleiner als 0,2 Millimeter, weiter bevorzugt von kleiner als 0,1 Millimeter auf.

Gemäß einer weiteren Ausführungsform ist der schlauchförmige Basiskörper aus einem faltbaren Material gefertigt.

Vorzugsweise ist der Basiskörper aus einem anderen Material gefertigt als die die Ankopplungsubstanz einschließenden Wandungen des Bodens. Das faltbare Material ist vorzugsweise nicht oder nur wenig elastisch verformbar. Unter "faltbar" ist vorliegend zu verstehen, dass der schlauchförmige Basiskörper zusammenklappbar oder zusammenlegbar ist und sich aus einem zusammengefalteten Zustand nicht selbsttätig in einen auseinandergefalteten Zustand verformt. Der Boden kann mit dem Basiskörper beispielsweise verklebt oder verschweißt sein.

Gemäß einer weiteren Ausführungsform ist das faltbare Material eine Polyurethanfolie.

Das Polyurethanmaterial ist vorzugsweise nicht oder nur wenig elastisch verformbar. Alternativ kann das faltbare Material ein beliebig anderes elastisch nicht oder nur wenig verformbares Material sein.

Gemäß einer weiteren Ausführungsform ist der Boden rund oder rechteckig.

Vorzugsweise ist der Boden größer als eine Spitze des Sonografieschallkopfs. Hierdurch kann der Sonografieschallkopf auf dem Boden bewegt werden.

Gemäß einer weiteren Ausführungsform ist die Ankopplungssubstanz ein wasserbasiertes Sonografiegel.

Hierdurch sind Standard-Sonografiegele für die Herstellung des Überzugs verwendbar. Dadurch kann der Überzug kostengünstig hergestellt werden.

Weiterhin wird eine Vorrichtung mit einem Sonografieschallkopf eines medizinischen Sonografiegeräts und einem derartigen Überzug vorgeschlagen.

Die Vorrichtung kann ferner ein medizinisches Sonografiegerät mit einem Ultraschallgenerator aufweisen.

Gemäß einer Ausführungsform sind der Sonografieschallkopf vollständig und eine Kabelverbindung von dem Sonografieschallkopf zu einem Ultraschallgenerator des medizinischen Sonografiegeräts zumindest teilweise in dem Überzug aufgenommen.

Hierdurch sind der Sonografieschallkopf und die zugehörige Kabelverbindung steril in dem Überzug aufgenommen.

Ferner wird eine Verpackungseinheit mit einem derartigen Überzug und einem Verpackungselement vorgeschlagen, mit dem der Überzug verpackt ist.

Das Verpackungselement kann beispielsweise eine Blisterverpackung oder ein Folienbeutel sein.

Gemäß einer Ausführungsform ist der Überzug steril verpackt.

Hierdurch ist stets eine Sterilität des Überzugs gewährleistet. Vorzugsweise wird der Überzug erst unmittelbar vor Gebrauch, beispielsweise im Operationssaal, aus dem Verpackungselement entnommen.

Weitere mögliche Implementierungen der Erfindung umfassen auch nicht explizit genannte Kombinationen von zuvor oder im Folgenden bezüglich der Ausführungsbeispiele beschriebenen Merkmale oder Ausführungsformen. Dabei wird der Fachmann auch Einzelaspekte als Verbesserungen oder Ergänzungen zu der jeweiligen Grundform der Erfindung hinzufügen.

Weitere vorteilhafte Ausgestaltungen und Aspekte der Erfindung sind Gegenstand der Unteransprüche sowie der im Folgenden beschriebenen Ausführungsbeispiele der Erfindung. Im Weiteren wird die Erfindung anhand von bevorzugten Ausführungsformen unter Bezugnahme auf die beigelegten Figuren näher erläutert.
Fig. 1 zeigt eine schematische Ansicht einer Ausführungsform einer Vorrichtung;
Fig. 2 zeigt eine schematische Schnittansicht einer Ausführungsform eines Überzugs für die Vorrichtung gemäß der Fig. 1;
Fig. 3 zeigt eine schematische Schnittansicht einer weiteren Ausführungsform eines Überzugs für die Vorrichtung gemäß der Fig. 1;
Fig. 4 zeigt eine schematische Draufsicht des Überzugs gemäß der Fig. 2 und 3;
Fig. 5 zeigt eine schematische Ansicht des Überzugs gemäß der Fig. 3 in einem zusammengefalteten Zustand; und
Fig. 6 zeigt eine schematische Ansicht einer Verpackungseinheit.

In den Figuren sind gleiche oder funktionsgleiche Elemente mit denselben Bezugszeichen versehen worden, sofern nichts anderes angegeben ist.

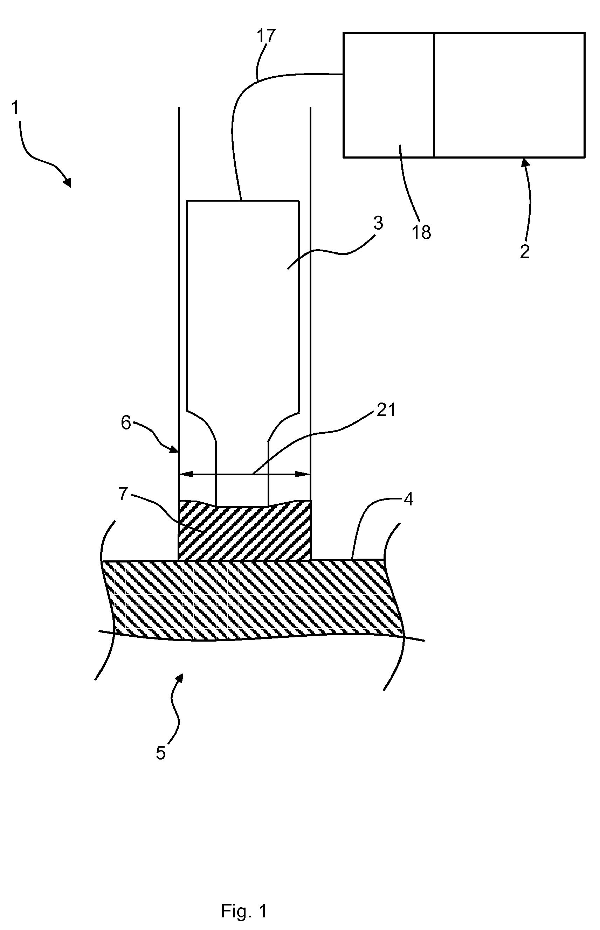

Die Fig. 1 zeigt eine schematische Ansicht einer Vorrichtung 1 für ein medizinisches Sonografiegerät 2. Die Vorrichtung 1 umfasst einen Sonografieschallkopf 3 des medizinischen Sonografiegeräts 2. Der Sonografieschallkopf 3 kann auch als Ultraschallsonde, Ultraschallkopf oder Transducer bezeichnet werden. Das medizinische Sonografiegerät 2 kann auch als Ultraschallgerät bezeichnet werden. Der Sonografieschallkopf 3 ist vorzugsweise stift- oder stabförmig. Der Sonografieschallkopf 3 kann einen runden oder eckigen Querschnitt aufweisen. Beispielsweise ist der Sonografieschallkopf 3 ein sogenannter Vaginalschallkopf mit einem runden Querschnitt und einem Durchmesser von 1,5 bis 2 Zentimetern. Der Sonografieschallkopf 3 kann alternativ eine rechteckige Geometrie mit einer beispielhaften Abmessung von 2 x 8 Zentimetern aufweisen. Der Sonografieschallkopf 3 ist insbesondere für einen intraoperativen Einsatz geeignet.

Der Sonografieschallkopf 3 ist dazu eingerichtet, mittels eines Piezoelements Ultraschallwellen zu erzeugen. Hierzu wird das Piezoelement mittels einer hochfrequenten elektrischen Wechselspannung zu Schwingungen angeregt. Das Piezoelement erzeugt dadurch Druckschwankungen in Form von Ultraschall. Der Sonografieschallkopf 3 ist auf einen zu untersuchenden Körperbereich 4 eines Patienten 5 auflegbar. Die von dem Sonografieschallkopf 3 ausgeschallten Ultraschallwellen werden je nach Art des beschallten Gewebes des Patienten 5 mehr oder weniger stark reflektiert. Die auf den Sonografieschallkopf 3 auftreffenden, vom Gewebe reflektierten Ultraschallwellen erzeugen in dem Piezoelement eine elektrische Spannung. Diese elektrische Spannung kann von dem medizinischen Sonografiegerät 2 als Bildpunkt dargestellt werden. Hierdurch lassen sich beispielsweise Organe des Patienten 5 darstellen.

Die Vorrichtung 1 weist neben dem Sonografieschallkopf 3 einen Überzug 6 auf. Der Überzug 6 umfasst eine Ankopplungssubstanz 7 zum Ankoppeln des Sonografieschallkopfs 3 an den zu untersuchenden Körperbereich 4 des Patienten 5. Die Ankopplungssubstanz 7 ist erforderlich, da der zu untersuchende Körperbereich 4 und sich möglicherweise zwischen dem Sonografieschallkopf 3 und dem zu untersuchenden Körperbereich 4 befindliche Luft unterschiedliche Impedanzen aufweisen. Unter "Impedanz" ist vorliegend der Widerstand zu verstehen, der der Ausbreitung der Ultraschallwellen entgegenwirkt. An einer Grenzfläche zwischen Luft und dem zu untersuchenden Körperbereich 4 werden die Ultraschallwellen bei Abwesenheit der Ankopplungssubstanz 7 stark reflektiert. Daher findet die Ankopplungssubstanz 7 Anwendung, die die Bildung von Lufteinschlüssen zwischen dem Sonografieschallkopf 3 und dem zu untersuchenden Körperbereich 4 verhindert. Die Ankopplungssubstanz 7 ist zwischen dem Sonografieschallkopf 3 und dem zu untersuchenden Körperbereich 4 angeordnet. Die Ankopplungssubstanz 7 ist vorzugsweise ein wasserbasiertes Sonografiegel. Weiterhin kann die Vorrichtung 1 das medizinische Sonografiegerät 2 aufweisen.

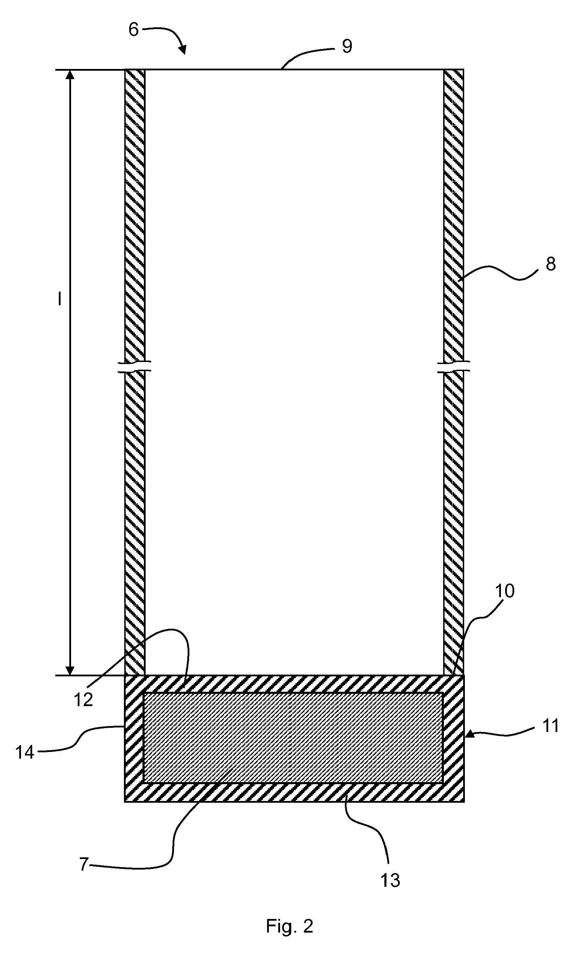

Die Fig. 2 zeigt eine schematische Schnittansicht des Überzugs 6. Der Überzug 6 weist einen schlauchförmigen oder rohrförmigen Basiskörper 8 auf, in dem der Sonografieschallkopf 3 aufnehmbar ist. Insbesondere ist der schlauchförmige Basiskörper 8 dazu eingerichtet, den Sonografieschallkopf 3 vollständig sowie eine Kabelverbindung 17 von dem Sonografieschallkopf 3 zu einem Ultraschallgenerator 18 des medizinischen Sonografiegeräts 2 zumindest teilweise aufzunehmen (Fig. 1). Der Basiskörper 8 weist vorzugsweise eine Länge I von 2 bis 3 Metern auf. Mit Hilfe des Basiskörpers 8 sind der Sonografieschallkopf 3 sowie die Kabelverbindung 17 steril verpackbar. Der schlauchförmige Basiskörper 8 weist insbesondere eine erste Stirnseite 9 und eine zweite Stirnseite 10 auf. Die Stirnseite 9 ist vorzugsweise offen, so dass durch die Stirnseite 9 der Sonografieschallkopf 3 in den Überzug 6 einführbar ist. Die Länge I des schlauchförmigen Basiskörpers 8 ist vorzugsweise so groß, dass die Stirnseite 9 desselben aus einem sterilen Feld herausführbar ist. Unter dem "sterilen Feld" ist bei einem Operationsvorgang der Bereich zu verstehen, der steril gehalten ist. Das medizinische Sonografiegerät 2 selbst ist vorzugsweise außerhalb des sterilen Felds angeordnet.

An der Stirnseite 10 des Basiskörpers 8 ist vorzugsweise ein die Stirnseite 10 verschließender Boden 11 vorgesehen. Der Boden 11 weist die Ankopplungssubstanz 7 auf. Die Ankopplungssubstanz 7 ist in den Boden 11 integriert. Vorzugsweise ist der Boden 11 kissenförmig. Das heißt, der Boden 11 umschließt die Ankopplungssubstanz 7 vollständig und weist hierzu eine erste Wandung 12 sowie eine zweite Wandung 13 auf, zwischen denen die Ankopplungssubstanz 7 angeordnet ist. Die erste Wandung 12 ist vorzugsweise beabstandet von und parallel zu der zweiten Wandung 13 angeordnet. Der Boden 11 weist ferner einen schlauch- oder rohrförmigen Verbindungsabschnitt 14 auf, der die erste Wandung 12 mit der zweiten Wandung 13 verbindet. Der Verbindungsabschnitt 14 und die Wandungen 12, 13 sind vorzugsweise aus einem anderen Material gefertigt als der Basiskörper 8.

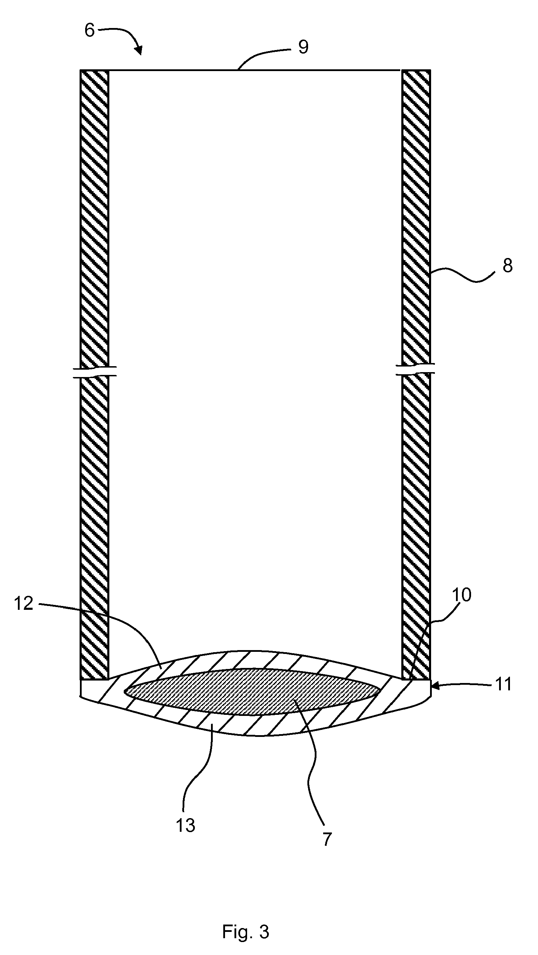

Alternativ zu der Anordnung der Wandungen 12, 13 parallel zueinander können die Wandungen 12, 13, wie die Fig. 3 zeigt, auch eine gekrümmte oder gebogene Form aufweisen. Hierdurch kann der Boden 11 eine linsenförmige Geometrie aufweisen. Bei dieser Ausführungsform mit gekrümmten Wandungen 12, 13 kann der Verbindungsabschnitt 14 verzichtbar sein. Alternativ kann auch nur eine der Wandungen 12, 13 gekrümmt sein.

Der schlauchförmige Basiskörper 8 ist vorzugsweise aus einem faltbaren, insbesondere aus einem elastisch nicht oder nur wenig verformbaren, Material gefertigt. Unter "faltbar" ist zu verstehen, dass der Basiskörper 8 zusammenlegbar oder zusammenklappbar ist und dass der Basiskörper 8 nach dem Zusammenfalten in dem zusammengefalteten Zustand verbleibt ohne dass sich der Basiskörper 8 selbsttätig in seine ursprüngliche Form zurückverformt. Der Basiskörper 8 kann beispielsweise aus einem Polyurethanwerkstoff gefertigt sein. Der schlauchförmigen Verbindungsabschnitt 14 und die Wandungen 12, 13 sind vorzugsweise aus einem elastisch verformbaren Material, insbesondere aus einem gummielastisch verformbaren Kunststoffmaterial, gefertigt. Das elastisch verformbare Material kann eine Latexfolie, eine Polyethylenfolie oder eine Silikonelastomerfolie sein.

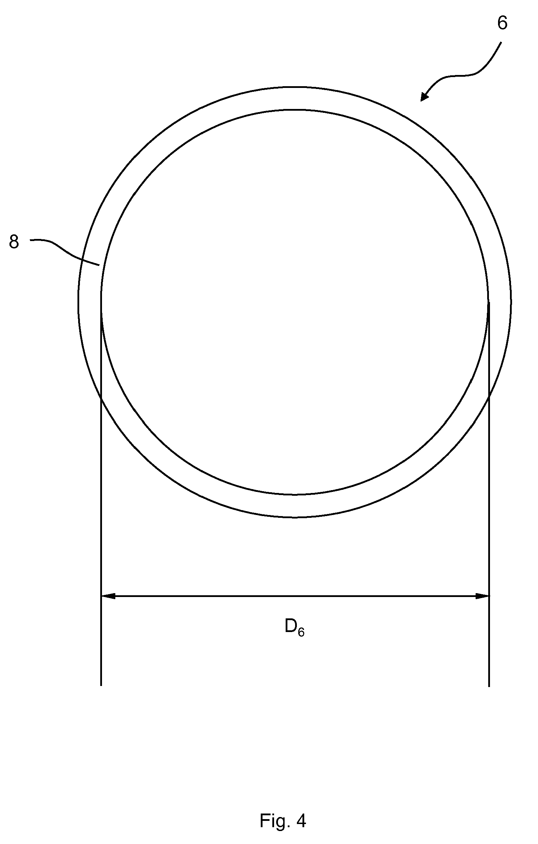

Der schlauchförmige Basiskörper 8 weist einen Innendurchmesser D₆ auf (siehe Fig. 4). Die Länge I beträgt vorzugsweise ein Vielfaches des Innendurchmessers D₆. Der Innendurchmesser D₆ des Überzugs 6 ist vorzugsweise größer als ein Außendurchmesser D₃ des Sonografieschallkopfs 3 (siehe Fig. 1). Das heißt, beim Überziehen des Überzugs 6 über den Sonografieschallkopf 3 kann zwischen dem Sonografieschallkopf 3 und dem Basiskörper 8 ein Luftspalt vorgesehen sein. Alternativ kann der Basiskörper 8 locker an dem Sonografieschallkopf 3 anliegen.

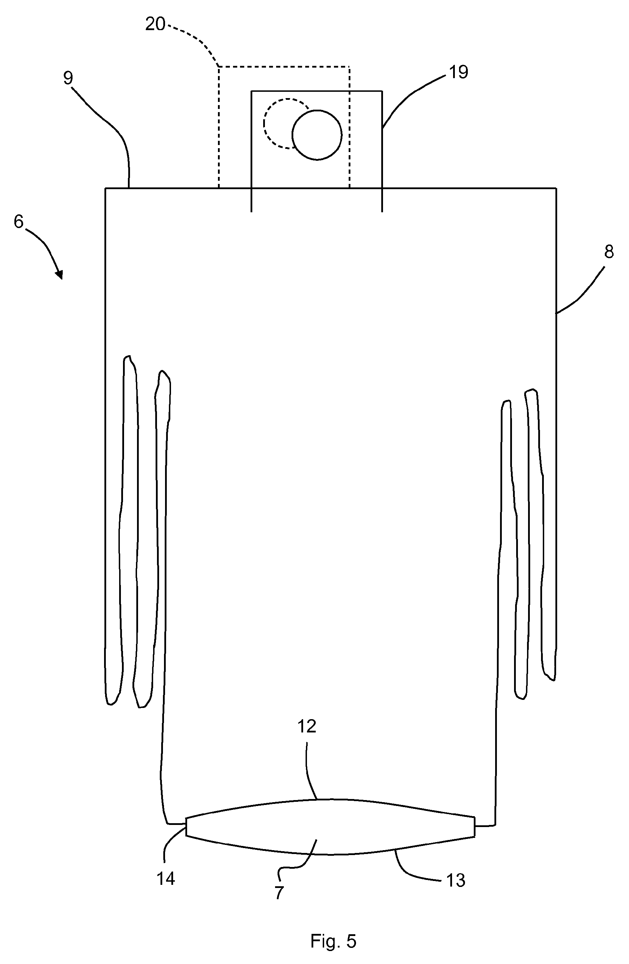

Die Fig. 5 zeigt den Überzug 6 in einem zusammengefalteten Zustand. Hierbei ist der schlauchförmige Basiskörper 8 ziehharmonikaartig zusammengefaltet. Der Überzug 6 weist zumindest einen Anfasser 19, 20 auf. Der Anfasser 19, 20 kann unsteril sein. Vorzugsweise sind zumindest zwei einander gegenüberliegend angeordnete Anfasser 19, 20 vorgesehen.

Das Anlegen des Überzugs 6 an den Sonografieschallkopf 3 wird wie im Folgenden erläutert durchgeführt. Ein Springer lässt zunächst den Sonografieschallkopf 3 in den zusammengefalteten sterilen Überzug 6 hineingleiten. Unter einem "Springer" ist in einem Operationssaal eine Person zu verstehen, die Sterilgut anreicht. Der Springer ist nicht steril. Eine den Sonografieschallkopf 3 bedienende, sterile Person fasst eine Spitze des sich in dem Überzug 6 befindlichen Sonografieschallkopfs 3. Der Springer fasst den Überzug 6 an einem der Stirnseite 9 des Basiskörpers 8 zugeordneten Endabschnitt. Vorzugsweise fasst der Springer den Überzug 6 an den Anfassern 19, 20 und zieht die Anfasser 19, 20 zum Entfalten des Basiskörpers 8 von dem sterilen Feld weg.

Wie die Fig. 1 zeigt, wird der Sonografieschallkopf 3 in den Boden 11 eingedrückt. Dadurch, dass die Wandungen 12, 13 elastisch, insbesondere gummielastisch, verformbar sind, kann der Sonografieschallkopf 3 auf dem Boden 11 hin und her bewegt werden. Diese Bewegbarkeit ist in der Fig. 1 mit einem Doppelpfeil 21 verdeutlicht.

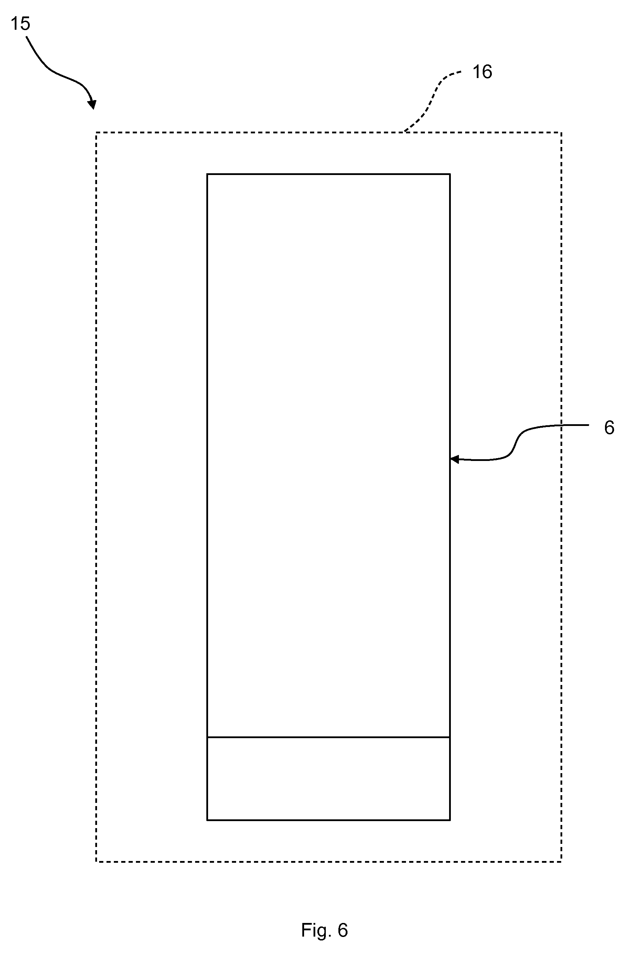

Die Fig. 6 zeigt eine Verpackungseinheit 15 mit einem derartigen Überzug 6 und einem Verpackungselement 16, in dem der Überzug 6 verpackt ist. Das Verpackungselement 16 kann beispielsweise eine Blisterverpackung oder ein Kunststoffbeutel sein. Insbesondere ist der Überzug 6 in dem Verpackungselement 16 steril verpackt. Der Überzug 6 wird vorzugsweise unmittelbar vor dem Gebrauch, beispielsweise während einer Operation, aus dem Verpackungselement 16 entnommen. Nach einmaligem Gebrauch wird der Überzug 6 entsorgt.

Obwohl die vorliegende Erfindung anhand von Ausführungsbeispielen beschrieben wurde, ist sie vielfältig modifizierbar.

### Verwendete Bezugszeichen:

- 1: Vorrichtung
- 2: Sonografiegerät
- 3: Sonografieschallkopf
- 4: Körperbereich
- 5: Patient
- 6: Überzug
- 7: Ankopplungssubstanz
- 8: Basiskörper
- 9: Stirnseite
- 10: Stirnseite
- 11: Boden
- 12: Wandung
- 13: Wandung
- 14: Verbindungsabschnitt
- 15: Verpackungseinheit
- 16: Verpackungselement
- 17: Kabelverbindung
- 18: Ultraschallgenerator
- 19: Anfasser
- 20: Anfasser
- 21: Pfeil

- D₃: Außendurchmesser
- D₆: Innendurchmesser
- I: Länge

## Patentansprüche

1. Überzug (6) für einen Sonografieschallkopf (3) eines medizinischen Sonografiegeräts (2), mit einem schlauchförmigen Basiskörper (8), in dem der Sonografieschallkopf (3) aufnehmbar ist, und einem eine Stirnseite (10) des Basiskörpers (8) verschließenden Boden (11), wobei der Boden (11) eine Ankopplungssubstanz (7) zum Ankoppeln des Sonografieschallkopfs (3) an einen zu untersuchenden Körperbereich (4) eines Patienten (5) aufweist.

2. Überzug nach Anspruch 1, **dadurch gekennzeichnet, dass** der Boden (11) kissenförmig ist.

3. Überzug nach Anspruch 1 oder 2, **dadurch gekennzeichnet, dass** die Ankopplungssubstanz (7) in den Boden (11) integriert ist.

4. Überzug nach einem der Ansprüche 1 - 3, **dadurch gekennzeichnet, dass** der Boden (11) eine erste Wandung (12) und eine beabstandet von der ersten Wandung (12) angeordnete zweite Wandung (13) aufweist.

5. Überzug nach Anspruch 4, **dadurch gekennzeichnet, dass** die Ankopplungssubstanz (7) zwischen der ersten Wandung (12) und der zweiten Wandung (13) angeordnet ist.

6. Überzug nach Anspruch 4 oder 5, **dadurch gekennzeichnet, dass** die Wandungen (12, 13) aus einem elastisch verformbaren Material gefertigt sind.

7. Überzug nach Anspruch 6, **dadurch gekennzeichnet, dass** das elastisch verformbare Material eine Polyethylenfolie oder eine Silikonelastomerfolie ist.

8. Überzug nach einem der Ansprüche 1 - 7, **dadurch gekennzeichnet, dass** der schlauchförmige Basiskörper (8) aus einem faltbaren Material gefertigt ist.

9. Überzug nach Anspruch 8, **dadurch gekennzeichnet, dass** das faltbare Material eine Polyurethanfolie ist.

10. Überzug nach einem der Ansprüche 1 - 9, **dadurch gekennzeichnet, dass** der Boden (11) rund oder rechteckig ist.

11. Überzug nach einem der Ansprüche 1 - 10, **dadurch gekennzeichnet, dass** die Ankopplungssubstanz (7) ein wasserbasiertes Sonografiegel ist.

12. Vorrichtung (1) mit einem Sonografieschallkopf (3) eines medizinischen Sonografiegeräts (2) und einem Überzug (6) nach einem der Ansprüche 1 - 11.

13. Vorrichtung nach Anspruch 12, **dadurch gekennzeichnet, dass** der Sonografieschallkopf (3) vollständig und eine Kabelverbindung (17) von dem Sonografieschallkopf (3) zu einem Ultraschallgenerator (18) des medizinischen Sonografiegeräts (2) zumindest teilweise in dem Überzug (6) aufgenommen sind.

14. Verpackungseinheit (15) mit einem Überzug (6) nach einem der Ansprüche 1 - 11 und einem Verpackungselement (16), mit dem der Überzug (6) verpackt ist.

15. Verpackungseinheit nach Anspruch 14, **dadurch gekennzeichnet, dass** der Überzug (6) steril verpackt ist.
